# EUROPEAN PATENT SPECIFICATION

(11) **EP 0 311 219 B1**
(45) Date of publication and mention of the grant of the patent: **14.08.1996**
(21) Application number: 88202248.6
(22) Date of filing: 07.10.1988
(51) Int. Cl.: C07K 7/06, C07K 7/08, G01N 33/68, A61K 38/08, A61K 39/21, A61K 39/395, G01N 33/569

(54) **Oligopeptides and their use for diagnostic and vaccination purposes for AIDS and ARC**
Oligopeptide und ihre Anwendung zu Diagnose- und Impfzwecken für AIDS und ARC
Oligopeptide et leur application pour le diagnostic et la vaccination relatifs au SIDA et â l'ARC

(30) Priority: 09.10.1987 NL 8702403
(43) Date of publication of application: 12.04.1989
(73) Proprietor: STICHTING CENTRAAL DIERGENEESKUNDIG INSTITUUT, NL-8219 PH Lelystad (NL); ACADEMISCH ZIEKENHUIS BIJ DE UNIVERSITEIT VAN AMSTERDAM, NL-1105 AZ Amsterdam (NL)
(72) Inventor: Goudsmit, Jaap, NL-1012 ES Amsterdam (NL); Meloen, Robert Hans, NL-8231 AP Lelystad (NL)
(74) Representative: Smulders, Theodorus A.H.J., Ir.

(56) References cited:
- WO-A-86/02383
- WO-A-87/02775
- CHEMICAL ABSTRACTS, vol. 106, 1987, page 469, no. 16942b, Columbus, Ohio, US; & US-A-779 431 (UNITED STATES DEPT. OF HEALTH AND HUMAN SERVICES) 14-03-1986
- CHEMICAL ABSTRACTS, vol. 106, 1987, page 506, no. 100556m, Columbus, Ohio, US; S. MODROW et al.: "Computer-assisted analysis of envelope protein sequences of seven human immunodeficiency virus isolates: prediction of antigenic epitopes in conserved and variable regions", & J. VIROL. 1987, 61(2), 570-8
- CHEMICAL ABSTRACTS, vol. 107, 1987, page 485, no. 113939j, Columbus, Ohio, US; V. KRCHNAK et al.: "Computer prediction of potential immunogenic determinants from protein amino acid sequence", & ANAL. BIOCHEM. 1987, 165(1), 200-

## Description

This invention relates to oligopeptides suitable for use in the diagnosis of, and vaccination against, AIDS (Acquired Immunodeficiency Syndrome) and ARC (AIDS-Related Complex) and, in addition to the new oligopeptides themselves, relates to their use for diagnostic purposes and vaccination compositions containing the new oligopeptides.

It is well known that AIDS and ARC are caused by a retrovirus, and that there are many variants of the virus. Various names are used for the virus, such as HTLV-III (human T lymphotropic virus type III), HIV (human immunodeficiency virus), LAV (lymphadenopathy-associated virus) and ARV (AIDS-associated retrovirus). It is found that many patients with AIDS or ARC form specific antibodies against proteins of the virus, especially against antigenic determinants located on the envelope glycoprotein gp120. However, the virus proves to be capable of avoiding the virus neutralizing effect of antibodies by changing certain parts of the gp120. In the envelope protein gp120, 5 areas have been indicated which exhibit a strong variation of the amino acids sequence between different variants of the virus. As regards these variations, reference is made to "Human Retroviruses and AIDS, A Compilation and Analysis of Nucleic Acid and Amino Acid Sequences", eds. Myers, Josephs, Rabson, Smith (1987).

After infection with the AIDS virus, human beings and also experimentally infected chimpanzees develop antibodies against the exterior envelope protein gp120. These antibodies are demonstrable soon after infection, and continue to circulate irrespective of the clinical condition. Antibodies neutralizing the prototype AIDS virus HTLV-III, measured by means of a CD4-dependent cell fusion inhibition test or HIV replication inhibition test, do not occur until later. People developing these virus neutralizing antibodies appear to remain healthy for a longer period of time than do people doing this to a lesser extent. The recognition of parts of gp120 by immune globulines of Man is associated with the virus neutralizing capacity of such sera. In this connection it has been demonstrated that goat sera directed against a recombinant protein PBI (DNA sequence PvuII - 2d Bg1II restriction site) neutralize the homologous virus (Putney et al., Science 234, (1986) 1392-1396) and the mouse sera against two synthetic peptides (amino acid 458-484/503-532 and 298-314) located within this recombinant protein also have virus neutralizing activity (Ho et al. J. Virol 61 (1987) 2024-2028). However, no oligopeptides of gp120 have been described which are immunogenic in the natural or experimental host (Man and chimpanzee) and may or may not be associated with the possible induction of virus neutralization. A knowledge of such short amino acid sequences is of interest to diagnostics and vaccine development.

Modrow et al. in J. Virol. 61(2), Feb. 1987, p. 570-578, teach the position, as predicted by computer analysis, of potential antigenic sites in the proteins gp120 and gp41. The outcome of the computer analysis, which predicts a secondary protein structure based on values of hydrophilicity, flexibility, surface probability and glycosylation, is a total of eleven potential antigenic sites, 9 in gp120 and 2 in gp41. One of the 9 potential antigenic sites in gp120 is epitope IV in the V3 region. This epitope comprises the amino acids 300 to 320 (numbering of isolate WMJ1).

If these epitope data are considered to suggest oligopeptides, these would have a length of about 21 or 22 amino acids (epitope IV comprises amino acids 300-321 in isolate BH10, i.e. 22 amino acids). Moreover, oligopeptides based on epitope IV as suggested by Modrow et al. would have the β-turn sequence GPGR very close to one end (the right end of the epitope is GPGRA).

Modrow et al. merely predict antibody binding sites (epitopes) on gp120 and gp41. They do not show which epitopes have a role in eliciting or binding virus-neutralizing antibodies and protective immune responses.

The international patent application W087/02775 discloses oligopeptides based on the amino acid sequence of the gp41 and gp120 subunits of the gp160 envelope glycoprotein of the HIV virus. The peptides are based on results of computer modeling of the structure of the gp120 and gp41 subunits. On the basis of the computer modeling, 6 sequences were selected for the preparation of synthetic peptides. These peptides were used to produce antibodies which are able to recognize native proteins associated with the HIV virus.

Only one of the 6 peptides shown (peptide No. 2) is based on the V3 region of gp120. Said peptide has the following amino acid sequence (using the one-letter code): TRPNNNTRKSIRIQRGPG. This peptide has a length of 18 amino acids and the β-turn sequence is located at the end (the last three amino acids are gly pro gly, i.e. GPG).

It is not shown which peptides, if any, induce antibodies which are capable of neutralizing the viral causative agents of AIDS and ARC and, actually, none of them has the property of inducing neutralizing antibodies.

The international patent application W086/02383 discloses envelope antigens of lymphadenopathy associated virus (LAV), including a glycoprotein having a molecular weight of about 110,000 and specific peptides derived therefrom. One of said peptides is derived from the amino acids 300-327 of said glycoprotein and consists of the amino acid sequence (using the one-letter code): INCTRPNNNTRKSIRIQRGPGR. It consists of 28 amino acid residues and has the β-turn sequence at the end.

By using the PEPSCAN method of Geysen et al. (Proc. Natl. Acad. Sci. USA 81 (1984) 3998-4002; Proc. Natl. Acad. Sci. USA 82 (1985) 178-182; "Synthetic peptides as antigens" 1986, 130-149, Wiley; WO84/03506 and WO84/03564) and polyclonal sera of HIV infected human beings and chimpanzees, we have now localized the sequence 305-321 (in the amino acids numbering of HTLV-III_{B} (BH10)) on the gp120 of the virus in the variable region V₃. It has been found that this sequence is an immunodominant epitope for neutralizing antibodies of infected human beings and chimpanzees, that is to say, the natural hosts, and that antibodies generated by a given variant of the virus, such as the BH10 variant, do react with the BH10 sequence 305-321, but not with the corresponding sequence of other variants, such as the RF variant, and the

other way round.

The sequence referred to comprises the amino acids sequence GPG or GPGR, responsible for a β-turn in the protein structure. This β-turn sequence is necessary for the protein structure and therefore highly conserved, but not responsible for immunogeneity, in view of the fact that sera specific for, for example, BH10, do not react with, for example, RF, and the other way round; cross reactions do not occur. The specific immunogeneity is therefore evidently caused by the flanking amino acid sequences.

This understanding is used, according to the present invention, by providing oligopeptides composed of 8-17 amino acids in a sequence corresponding with a sequence occurring in the envelope protein gp120 of an AIDS or ARC causing or related virus in the variable V₃ area, consisting of the β-turn amino acids sequence GPG or GPGR at the positions 312-314 or 312-315 in the amino acids numbering of HTLV-III_{B} (BH10) and flanking amino acids sequences at least 1, preferably at least 2 amino acids long, and variants thereof in which the GPG or GPGR sequence has been replaced by a different β-turn sequence, and variants in which the free amino group of the amino terminal amino acid and/or the free carboxyl group of the carboxy-terminal amino acid is blocked or otherwise modified.

These oligopeptides can be used for diagnostic purposes, in particular for determining, by means of a body fluid, such as serum of mammals, such as humans or chimpanzees, infection with an AIDS or ARC causing or related virus, and for determining the variant or variants of the virus with which the mammal has been infected, or which have formed during the infection, using known per se methods for detecting the binding of the antibodies present in the sample being investigated to the oligopeptides. Also, the new oligopeptides according to the new invention can be used for preparing vaccine preparations protecting against AIDS or ARC causing or related viruses.

In this specification, the amino acids are designated by the one-letter code, that is to say that, for example, G stands for glycine, P stands for proline, R stands for arginine, etc.

As the β-turn sequence GPG or GPGR is only of importance for the protein structure and not for the specific immunogeneity, the oligopeptides according to the invention may comprise a different β-turn sequence instead of the natural sequence GPG or GPGR. Examples of other β-turn sequences are GLGQ and GLGK, which occur in the gp 120 of various African HIV-1 strains. Such variants are therefore comprised by the invention.

In view of the contemplated uses, it may further be desirable that the terminal amino acids be modified. Possibilities in this connection are blocking the terminal free amino and carboxyl groups, or chemically modifying these groups otherwise. By way of example, the free amino group of the amino terminal amino acid could be acetylated and the free carboxyl group of the carboxy terminal amino acid could be amidated. Such variants are therefore also comprised by the present invention.

The oligopeptides according to the invention have a length of 8-17 amino acids, preferably 9-15, and most preferably 10-13 amino acids. The β-turn sequence is flanked by amino acid sequences having a length of at least 1 and preferably at least 2 amino acids each, preferably at least 3 and most preferably at least 4 amino acids.

Examples of oligopeptides according to the invention from 17 amino acids are:

KSIRIQRGPGRAFVTIG
KKIRIQRGPGRAFVTIG
VRKSITKGPGRVIYATG
ISQSIRIGPGKVFYAKG
VRRSLSIGPGRAFRTRE
RRRHIHIGPGRAFYTGE
ARRRIHIGPGRAFYTGK
VRKSIYIGPGRAFHTTG
RKSIIQRGPGRAFVTIG

Examples of shorter oligopeptides according to the invention are:

IRIQRGPGRAFVTIG
IQRGPGRAFVTIG
IQRGPGRAFVT
ITKGPGRVIYATG
ITKGPGRVIY
ITKGPGRVI
IQRGPGRAFV
IRIQRGPGRAF
TKGPGRVIYA

Methods of producing oligopeptides are generally known, so that their preparation will not be described in any detail herein.

The new oligopeptides according to the invention can be used for diagnostic purposes. In particular, one possibility is an examination of infected human of chimpanzee sera by means of a set of oligopeptides according to the invention to determine which variant of the virus has caused the infection and/or what new variants have formed during the infection. An insight into this is of diagnostic, prognostic and therapeutic interest. Techniques suitable for such examinations are known per se. By way of example we can mention here ELISA'S, RIPA'S, dot-blots, etc. According to a more concrete example, serum from an infected person can be contacted with a nitrocellulose strip on which different oligopeptides according to the invention have been arranged. By washing, adding an anti-hIg-antibody coupled to a peroxydase, re-washing and adding peroxidase substrate, colour formation can be obtained in the site of the oligopeptide with which the antibodies present in the sample react.

The new oligopeptides according to the invention also open the way for vaccines against AIDS and ARC, in particular multivalent vaccines or vaccine cocktails directed against a plurality of variants of the virus. The vaccin compositions according to the invention comprise one or (preferably) a plurality of oligopeptides according to the invention, in combination with one or a plurality of carriers and/or adjuvants suitable for vaccination purposes. Carriers and adjuvants suitable for vaccines are known per se. By way of example, we mention the possibility of coupling the oligopeptide by means of a suitable coupling agent to KLH (Keyhole Limpet Hemocyanin) or BSA (Bovine Serum Albumine). Toxoids and liposomes are also suitable carriers, and so are poly-L-lysine, poly-L-glutanic acid, muramyl dipeptide, murabutidine, etc. Suitable adjuvants are, for example, aluminium hydroxide and other known adjuvants. Diluents suitable for the administration of the vaccine, such as distilled water, phosphate-buffered saline, and buffer solutions (such as a citrate buffer) are also known per se.

The particular properties of the oligopeptides according to this invention, and thus their particular suitability for use in the above applications, have been confirmed by the results of a large number of experiments. Thus, for example, in a study of monoclonal antibodies, which had been established to neutralize the HIV-1 strain HTLV-IIIB, it was found that all antibodies investigated bind to oligopeptides according to the invention. A monoclonal antibody with a good virus neutralizing capacity was found to bind to an octapeptide IQRGPGRA, and another monoclonal antibody with a pronounced virus neutralizing capacity was found to bind to the octapeptide QRGPGRAF.

In a study of polyclonal antibodies, produced in a goat in response to recombinant gp160 of HTLV-IIIB and of HTLV-IIIRF, expressed in baculo virus, the antiserum against recombinant gp160-IIIRF, for example, was found to bind most strongly to the nonapeptide ITKGPGRVI from the V₃ region of HTLV-IIIRF, whereas it did not bind to the corresponding nonapeptide IQRGPGRAF of HTLV-IIIB. Similar results were obtained with polyclonal antibodies produced in a rabbit in response to the carboxyterminal portion of gp120 expressed in E. coli. All polyclonal antibodies neutralizing the homologue strain were found to bind to an oligopeptide according to the invention.

All these studies taken together justify the conclusion that antibodies produced by the immunization of, for example, mice, rabbits and goats with all sorts of potential vaccine products derived from the HIV-1 envelope precursor gp160, or the external envelope gp120, and exhibiting strain-specific HIV-1 neutralization in vitro, always bind around the β-turn sequence, i.e., to an oligopeptide according to this invention. Neutralizing sera of chimpanzees and humans also bind in the area in question, as has also been determined by experimentation.

The experiments were not only carried out with octa- and nonapeptides, but, for example, also with the 17 amino acids long HTLV-IIIB oligopeptide KSIRIQRGPGRAFVTIG, which bound human sera inhibiting cell fusion and neutralizing cell-free virus, and also bound goat antibodies against gp120 and against the carboxyterminal part of gp120, capable of neutralization and cell-fusion inhibition. Rabbit antibodies against this oligopeptide bound the HTLV-IIIB gp-120 and its precursor gp-160, while they also neutralized HTLV-IIIB and exhibited cell-fusion inhibiting properties.

The experiments conducted covered not only oligopeptides of various lengths, but also oligopeptides with widely divergent amino acid sequences, such as nonapeptides having the following sequences:

IQRGPGRAF (LAV-1)
IYIGPGRAF (SF-2)
ITKGPGRVI (RF)
IAIGPGRTL (NY-5)
VTLGPGRVW (CDC-4)
IHFGPGQAL (MAL)
IRIGPGKVF (Z3)
TPIGLGQAL (Z6)
TPIGLGQSL (ELI)

The codes in brackets indicate the virus strains from which the sequences have been derived. Human sera collected in Europe turned out to recognize especially nonapeptides derived from European strains, whereas peptides derived from African strains (ELI, MAL, Z3, Z6) and Haitian strains (RF) were recognized to a much lesser extent. Sera collected in Africa, on the other hand, were better in recognizing the peptides derived from African strains. Each individual tested turned out to have a different, individual pattern of oligopeptides recognition.

The experiments also confirmed the suitability of oligopeptides according to the invention as components of vaccine cocktails. In fact, virtually all sera investigated were found to recognize at least one oligopeptide according to the invention. By determining the amino acid sequence of gp120, or at least its relevant part in the V₃ region, of mutants of the virus, valuable new components of such vaccine cocktails can be designed. Although the length of the oligopeptides to be used can vary from 8 or 9 to 17 amino acids, a length of about 12 amino acids will usually be selected, because such a length appears to give an optimum specificity and virus neutralizing activity.

The experiments also confirm the suitability of oligopeptides according to the invention for diagnostic purposes. Thus the invention could be used in the form of ELISA plates with a panel of oligopeptides according to the invention thereon, for tracing the spread of HIV-strains with variation in the neutralizing epitope by determining the reactivity of the serum of an infected individual. The invention could also be used in the form of a set of antisera against a panel of oligopeptides according to this invention, so that viruses can be typed by means of RIPA.

### Example I: Synthesis of acetyl-Ile-Gln-Arg-Gly-Pro-Gly-Arg-Ala-Phe-NH2

Solvents and reagents were pro-analyse grade and were purchased from Merck, Darmstadt, GFR. They were used without further purification; dichloromethane (DCM) was purified over a column of activated aluminum oxide. Boc-amino acids, 4-methylbenzhydrylamine (MBHA) resin and benzotriazole-1-yl-oxy-tris-(dimethylamino)-phosphonium-hexa fluorophosphate (BOP, Castro's reagent) were purchased from Novabiochem, Laufelfingen, Switzerland. The side-chain of Arg was protected by the Tosyl group. The reactions were carried out in a glass vessel with a glass frit in a mechanically operated shaker. Routine liquid volumes were 7 ml.

Amino acid analyses were carried out with the Waters Pico-tag amino acid analysis system, after hydrolysis of the peptide in 1 % phenol in 6 N HCl at 150° for 1 hr. HPLC was carried out on an analytical reverse phase column (polygosil 60-10C18, 250 x 4 mm) using a Waters HPLC system consisting of a model 680 gradient controller and two model 510 pumps and a model WISP 712 automatic injector. Using a solvent system composed of eluent A, 20 % methanol/water (v/v) with 0.1 % trifluoroacetic acid (TFA), and eluent B, 80 % methanol/water with 0.1 % TFA, and at a flow rate of 1 ml/min the peptide was detected at 210 nm using a Spectroflow 757 spectrophotometer.

After 24 hr swelling in DCM, MBHA resin (1 % DVB, 0.5 meq/mg, 600 mg) was successively washed with DCM, 50 % TFA/DCM (v/v), DCM, 7 % diisopropylethylamine (DIEA)/DCM (v/v), and dimethylformamid (DMF).

Coupling procedure: To the MBHA resin, suspended in a minimal amount of DMF, was added 1 mmol of Boc-Phe in 1 ml of DMF. BOP (1 mmol in 1 ml of DMF) was added, directly followed by DIEA (3 mmol). After 1 hr the resin was successively washed with DMF (3 x 2 min), isopropanol (3 x 2 min), and DCM (3 x 2 min). The completeness of the coupling was examined by the Kaiser test (Anal. Biochem. 34, 1970, 594-598). If the reaction was not complete the coupling procedure was repeated. If the reaction was complete the Boc group was removed by reacting the resin with 50 % TFA/DCM (5 min), 50 % TFA/DCM (20 min), followed by washings with DCM (3 x 2 min), 7 % DIEA/DCM (3 x 4 min), and DMF (3 x 2 min). Then the next Boc-amino acid was coupled. At the end of the synthesis the N-terminal amino group was acetylated by acetic anhydride (1 ml in 3 ml of DMF and 0.5 ml of triethylamine) for 30 min. Yield: 908 ml. Deprotection: A part of the peptide resin (523 mg) was treated with 10 ml anhydrous HF containing 10 % anisole for 1 hr at 0°C. After removal of HF in vacuo, the crude reaction product was dissolved in 5 ml of 10 % acetic acid and the fluoride ions were exchanged on an ion-exchange resin (BioRad AG 2-X8; 3 x 1 cm, acetate form). The product was lyophilized from 1 % acetic acid. Yield: 154 mg. Amino acid analysis: Glu 0.96; Gly 2.00; Arg 2.23; Ala 0.96; Pro 0.95; Ile 0.83; Phe 0.91. Purity, according to HPLC, 70 %.

### Example II: Detection of antibodies to Neu21

The peptide KSIRIQRGPGRAFVTIG (Neu21) composed of amino acids 305-321 of the envelope gp120 of clone BH10 HTLV-IIIB was produced using the Merrifield solid phase synthesis.

The presence of antibodies was determined using a direct non-competitive solid phase immunoassay. Wells of microtiter plates were incubated with 0.1 mol/l NaH₂PO₄ (pH 5.0) and 0.2 % (volume/volume) glutardialdehyde (Merck, Meppel, the Netherlands) at a volume of 100 µ1 per well and incubated for four hours at room temperature. Subsequently, the plates were rinsed three times for 10 minutes with 0.1 mol/l NaH₂PO₄ (pH 5.0). Then 10 ng peptide per well was incubated in 0.1 mol/l Na₂HPO₄ (pH 8.0), 100 µl/well, for sixteen hours at room temperature. After coating, wells were washed ten times with PBS Tween-20 (0.1 % volume/volume). Non-specific binding sites were blocked by incubating for one hour at 37°C using 4 % normal goat serum (NGS) in PBS-Tween-20. Sera to be tested were diluted 1:100 in PBS-Tween-20, 4 % NGS and incubated for one hour at 37°C. After another washing step, horseradish peroxidase-labelled conjugates (goat antihuman IgG, KPL, Gaithersburg, Maryland, USA) were added, diluted 1:500 in PBS-Tween-20 with 4 % NGS. Bound antibodies were visualized using ophenylene diamine and the reaction was stopped with 1 N H₂SO₄. Optical density was read at an absorbance of 450 nm.

Interassay variation was controlled by including a serial dilution of a positive human control serum on each plate. The mean optical density of 149 HIV-1 seronegative samples plus four times the standard deviation was used as cut-off value.

## Claims

1. Oligopeptides composed of 8-17 amino acids in a sequence corresponding to a sequence occurring in the variable region V₃ in the envelope protein gp120 of an AIDS or ARC causing or related virus, comprising the β-turn amino acids sequence GPG or GPGR at the positions 312-314 312-315 in the amino acids numbering of HTLV-IIIB (BH10) and flanking amino acids sequences having a length of at least 1 and preferably at least 2 amino acids, and variants in which the GPG or GPGR sequence has been replaced by a different β-turn sequence, and variants in which the free amino group of the amino terminal amino acid and/or the free carboxyl group of the carboxy terminal amino acid has been blocked or modified otherwise.

2. Oligopeptides as claimed in claim 1, composed of 9-15 amino acids.

3. Oligopeptides as claimed in claim 1, composed of 10-13 amino acids.

4. Oligopeptides as claimed in claim 1, comprising the β-turn sequence GPG or GPGR and flanking amino acids sequences having a length of at least 3 amino acids.

5. Oligopeptides as claimed in claim 1, comprising the β-turn sequence GPG or GPGR and flanking amino acids sequences having a length of at least 4 amino acids.

6. Antibodies (monoclonal or polyclonal) raised against oligopeptides as claimed in any of claims 1-5.

7. The use of one or more oligopeptides as claimed in claim 1, or of one or more antibodies raised against oligopeptides as claimed in claim 1, for determining, by means of a body fluid, such as serum, of mammals, such as Man or chimpanzee, infection with an AIDS or ARC causing or related virus, and for determining the variant or variants of the virus with which the mammal is infected, or which have been formed during the infection, using known per se methods for detecting the binding of antibodies present in the sample being investigated to the oligopeptides or the binding of gp120 present in the sample to the antibodies.

8. A vaccine composition for protecting mammals, such as Man and chimpanzee, against AIDS or ARC causing or related viruses, comprising one or more oligopeptides as claimed in claim 1 and one or more carriers and/or adjuvants suitable for vaccination purposes.

9. A diagnostic aid for use in testing HIV contaminations and/or typing viruses causing AIDS or ARC, or related viruses, comprising one or more oligopeptides as claimed in claim 1 or one or more antibodies induced against oligopeptides as claimed in claim 1.

## Patentansprüche

1. Oligopeptide aus 8 - 17 Aminosäuren mit einer Sequenz, die der in der variablen Region V₃ des Hüllproteins gp120 eines AIDS- oder ARC-verursachenden oder -verwandten Virus auftretenden Sequenz entspricht, welche die Aminosäuresequenz GPG oder GPGR des β-Turns in den Positionen 312 - 314 bzw. 312 - 315 in der Aminosäurenumerierung von HTLV-IIIB (BH10) sowie flankierende Aminosäuresequenzen in der Länge von mindestens einer Aminosäure und vorzugsweise von mindestens 2 Aminosäuren umfaßt, sowie Varianten, in denen die GPG- oder GPGR-Sequenz durch eine andere β-Turnsequenz ersetzt worden ist, und Varianten, in denen die freie Aminogruppe der aminoterminalen Aminosäure und/oder die freie Carboxylgruppe der carboxyterminalen Aminosäure blockiert oder anders modifiziert worden ist.

2. Oligopeptide gemäß Anspruch 1 aus 9 bis 15 Aminosäuren.

3. Oligopeptide gemäß Anspruch 1 aus 10 bis 13 Aminosäuren.

4. Oligopeptide gemäß Anspruch 1, welche die β-Turnsequenzen GPG oder GPGR und flankierende Aminosäuresequenzen in der Länge von mindestens 3 Aminosäuren enthalten.

5. Oligopeptide gemäß Anspruch 1, welche die β-Turnsequenzen GPG oder GPGR und flankierende Aminosäuresequenzen in der Länge von mindestens 4 Aminosäuren enthalten.

6. Antikörper (monoklonale und polyklonale), die gegen ein Oligopeptid gemäß einem der Ansprüche 1 bis 5 gezogen wurden.

7. Verwendung von einem oder mehreren Oligopeptiden gemäß Andspruch 1 oder von einem oder mehreren der gegen die Oligopeptide gemäß Anspruch 1 gezogenen Antikörper zum Nachweis einer Infektion von Säugern wie dem Menschen oder Schimpansen mit einem AIDS- oder ARC-verursachenden oder verwandten Virus mittels einer Körperflüssigkeit wie dem Serum und zum Nachweis der Variante oder der Varianten des Virus, mit dem der Säuger infiziert ist bzw. die sich im Verlauf der Infektion gebildet haben, unter Anwendung ansich zum Nachweis der Bindung eines in der getesteten Probe anwesenden Antikörpers an das Oligopeptid oder der Bindung des in der Probe vorhandenen gp120 an den Antikörper bekannter Verfahren.

8. Impfzubereitung zum Schutz von Säugern wie dem Menschen oder Schimpansen gegen AIDS- oder ARC-verursachende oder verwandte Viren, die eine oder mehrere der Oligopeptide gemäß Anspruch 1 und eine oder mehrere für die Zwecke der Impfung geeignete Träger und/oder Hilfsstoffe enthält.

9. Diagnositzierhilfe zur Verwendung beim Testen auf HIV-Kontaminationen und/oder zum Typisierung von AIDS- oder ARC-verursachenden oder verwandten Viren, die ein oder mehrere Oligopeptide gemäß Anspruch 1 oder ein oder mehrere gegen die Oligopeptide gemäß Anspruch 1 induzierte Antikörper enthält.

## Revendications

1. Oligopeptides constitués de 8 à 17 acides aminés dans une séquence correspondant à une séquence apparaissant dans la région variable V₃ de la protéine d'enveloppe gp120 d'un virus provoquant le SIDA ou le para-SIDA ou d'un virus associé, comprenant la séquence d'acides aminés de courbure β GPG ou GPGR en positions 312-314 ou 312-315 selon la numérotation des acides aminés de HTLV-IIIB (BH10) et des séquences d'acides aminés encadrantes ayant une longueur d'au moins 1 acide aminé et de préférence d'au moins 2 acides aminés, ainsi que les variants dans lesquels la séquence GPG ou GPGR a été remplacée par une séquence de courbure β différente et les variants dans lesquels le groupement amino libre de l'acide aminé amino-terminal et/ou le groupement carboxyle libre de l'acide aminé carboxy-terminal a été bloqué ou sinon modifié.

2. Oligopeptides selon la revendication 1 constitués de 9 à 15 acides aminés.

3. Oligopeptides selon la revendication 1 constitués de 10 à 13 acides aminés.

4. Oligopeptides selon la revendication 1 comprenant la séquence de courbure β GPG ou GPGR et les séquences d'acides aminés encadrantes ayant une longueur d'au moins 3 acides aminés.

5. Oligopeptides selon la revendication 1 comprenant la séquence de courbure β GPG ou GPGR et les séquences d'acides aminés encadrantes ayant une longueur d'au moins 4 acides aminés.

6. Anticorps (monoclonaux ou polyclonaux) produits contre les oligopeptides selon l'une quelconque des revendications 1 à 5.

7. Utilisation d'un ou plusieurs oligopeptides selon la revendication 1 ou d'un ou plusieurs anticorps produits contre les oligopeptides selon la revendication 1 pour la détermination, au moyen d'un liquide organique, tel que le sérum, de mammifères, tels que l'homme ou le chimpanzé, de l'infection par un virus provoquant le SIDA ou le para-SIDA ou un virus associé et pour la détermination du ou des variants du virus par lequel ou lesquels le mammifère est infecté, ou bien qui a ou ont été formé(s) lors de l'infection, en utilisant des procédés, connus en soi, de détection de la liaison des anticorps présents dans l'échantillon étudié aux oligopeptides ou de la liaison de la gp120 présente dans l'échantillon aux anticorps.

8. Composition de vaccin destinée à la protection des mammifères, tels que l'homme et le chimpanzé, contre les virus provoquant le SIDA ou le para-SIDA ou les virus associés, comprenant un ou plusieurs oligopeptides selon la revendication et un ou plusieurs véhicules et/ou adjuvants adaptés à des fins de vaccination.

9. Aide diagnostique à utiliser pour la recherche des contaminations par HIV et/ou pour le typage des virus provoquant le SIDA ou le para-SIDA ou des virus associés, comprenant un ou plusieurs oligopeptides selon la revendication 1 ou un ou plusieurs anticorps induits contre les oligopeptides selon la revendication 1.
